# EUROPEAN PATENT APPLICATION

(11) **EP 4 606 891 A1**
(43) Date of publication of application: **27.08.2025**
(21) Application number: 23880228.4
(22) Date of filing: 18.10.2023
(51) Int. Cl.: C12N 5/0786

(54) **MEDIUM COMPOSITION FOR INDUCING DIFFERENTIATION FROM STEM CELLS TO MACROPHAGES AND DIFFERENTIATION METHOD USING SAME**

(30) Priority: 18.10.2022 KR 20220134330
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: KANG, Eun Ju, Seoul 04595 (KR); LEE, Yeon Mi, Gwangju-si, Gyeonggi-do 12787 (KR)
(74) Representative: Vossius & Partner Patentanwälte Rechtsanwälte mbB
(86) International application number: PCT/KR2023/016170
(87) International publication number: WO 2024/085651

(57) **Abstract**

Provided is a culture medium composition for inducing differentiation from stem cells to macrophages. According to one aspect, the culture medium composition for inducing differentiation from stem cells to macrophages can increase the efficiency of differentiation to macrophages and improve the expression of macrophage-specific markers.

## Description

### Technical Field

The present disclosure relates to a culture medium composition for inducing differentiation from stem cells to macrophages.

### Background Art

Pluripotent stem cells are undifferentiated cells that can differentiate into all cells that make up the human body. Following the establishment of human embryonic stem cells (hESCs) in the late 1990s, research in the stem cell field formally began and has achieved remarkable progress since the groundbreaking achievement of creating induced pluripotent stem cells (iPSCs) in the mid-2000s. Furthermore, with the recent successful establishment of human somatic-cell nuclear transfer ESCs (hSCNT-ESCs), research in the stem cell field has become even more active.

Among the research fields using such stem cells, various studies are underway on methods to differentiate cells into desired types in cell culture, and particularly, the development of protocols that can differentiate them more efficiently is being pursued as a major task. Undifferentiated stem cells gradually lose their stem cell characteristics and acquire the unique characteristics of differentiated cells as they differentiate. In this process, various signaling molecules, for example, morphogens and growth factors, act sequentially and in complex ways according to developmental stages. These factors are added to the stem cell culture medium to induce differentiation.

Macrophages are cells responsible for innate immune responses and exist throughout the body. Most macrophages are adhesive, including dust cells, microglia, Kupffer cells, and Langerhans cells. When they recognize antigens, they either phagocytose them, secrete toxins to destroy them, or initiate immune responses by delivering antigens to lymphocytes. Macrophages also exist as undifferentiated monocytes in the blood, which can differentiate into dendritic cells or macrophages as needed, and various signaling molecules are involved in macrophage differentiation.

Thus, induced differentiation of pluripotent stem cells can yield desired cells, and there is a need to develop novel stem cell differentiation induction methods and differentiation induction compositions that can increase differentiation efficiency and yield of final cells.

Although recent research on hematopoietic stem cell or macrophage differentiation has been introduced, it has not yet been connected to functional and practical research. The Nishimura Y group first attempted to induce differentiation of mouse DC cells and macrophages from mouse pluripotent stem cells, and research on differentiation to macrophages is ongoing, but related research in Korea is insufficient, and differentiation efficiency is low and expression of macrophage markers is low when differentiating to macrophages.

### Disclosure of Invention

### Technical Problem

One aspect provides a culture medium composition for inducing differentiation from stem cells to macrophages, including: (i) a first culture medium composition for inducing differentiation from stem cells to hematopoietic stem cells, including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof; (ii) a second culture medium composition for inducing differentiation from hematopoietic stem cells to macrophages, including one or more selected from the group consisting of a stem cell factor (SCF), Dickkopf-related protein 1 (DKK1), interleukin 3 (IL-3), fasudil, and combinations thereof; and (iii) a third culture medium composition for macrophage maturation, including one or more selected from the group consisting of a colony stimulating factor 1 (CSF-1), fasudil, and combinations thereof.

Another aspect provides a method of inducing differentiation from stem cells to macrophages, including: inducing differentiation from stem cells into hematopoietic stem cells in the presence of the first culture medium composition; inducing differentiation of hematopoietic stem cells to macrophages in the presence of the second culture medium composition; and maturing the macrophages in the presence of the third culture medium composition.

### Solution to Problem

One aspect provides a culture medium composition for inducing differentiation from stem cells to macrophages, including: (i) a first culture medium composition for inducing differentiation from stem cells to hematopoietic stem cells, including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof; (ii) a second culture medium composition for inducing differentiation from hematopoietic stem cells to macrophages, including one or more selected from the group consisting of a stem cell factor (SCF), Dickkopf-related protein 1 (DKK1), interleukin 3 (IL-3), fasudil, and combinations thereof; and (iii) a third culture medium composition for macrophage maturation, including one or more selected from the group consisting of a colony stimulating factor 1 (CSF-1), fasudil, and combinations thereof.

The term "macrophage" in this specification may refer to a type of tumor suppressor cell and a cell responsible for immunity.

The macrophages may exhibit the effect of removing cancer-associated cells from the cancer microenvironment. It is known that cancer-associated cells affect tumor growth, tumor angiogenesis, tumor cell invasion, and metastasis by promoting interactions within the cancer microenvironment, thereby increasing the malignancy of cancer cells and increasing the invasion of cancer cells. Therefore, by utilizing the macrophages, such macrophages can be utilized as an adjuvant for anticancer drugs or immunotherapy.

In addition, since the macrophages can directly kill and suppress cancer cells, the macrophages can be used as an anticancer agent.

The term "CHIR99021" of the present disclosure refers to a compound having the structure of Formula 1.

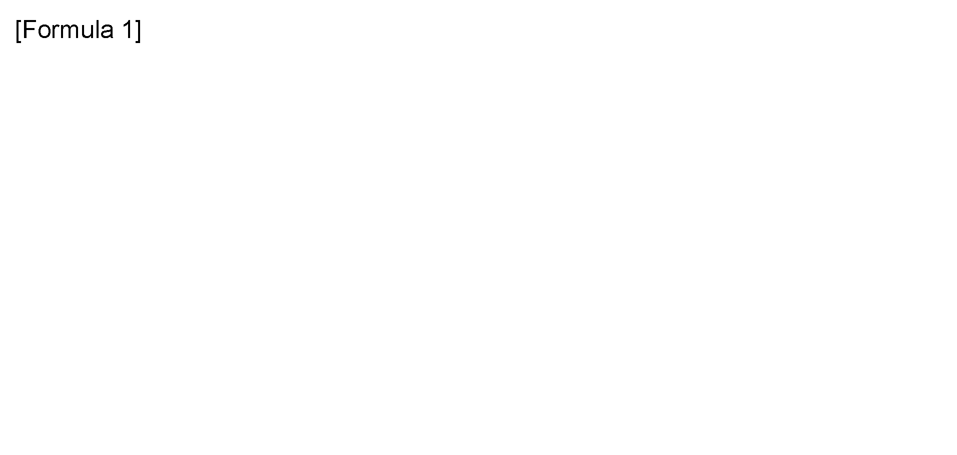

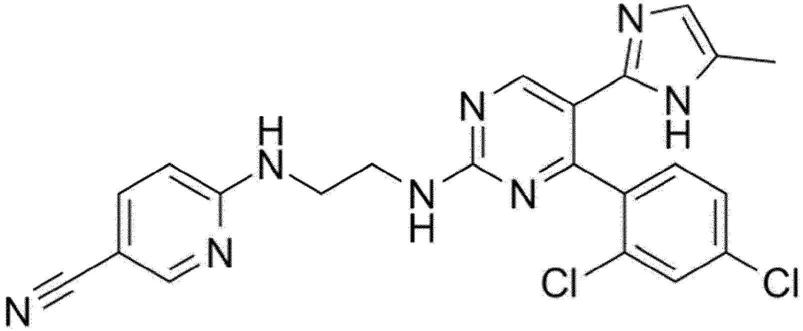

The term "SB-431542" of the present disclosure refers to a compound having the structure of Formula 2.

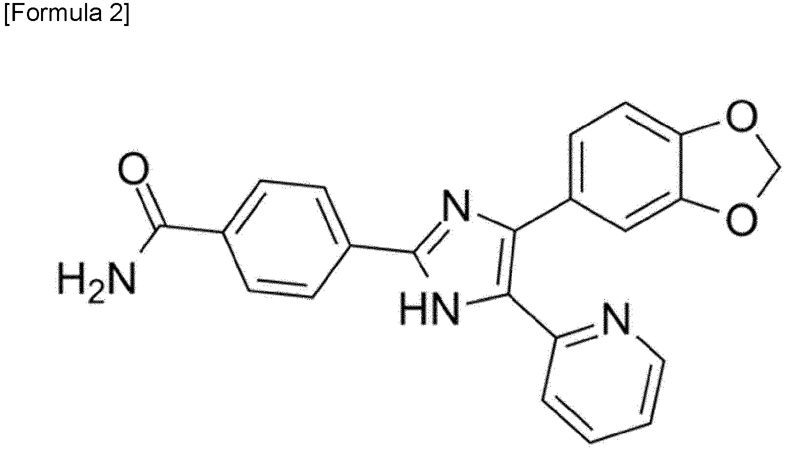

The "medium" as used herein may vary depending on the intended use, but may include all common media well known to those skilled in the art, including a medium for culturing animal cells that basically includes inorganic salts, carbon sources, amino acids, bovine serum albumin (BSA), and auxiliary factors. The medium may include inorganic salts such as NaCl, KCI, and NaHCO3, carbon sources such as glucose, sodium pyruvate, and calcium lactate, amino acids such as essential amino acids and nonessential amino acids, including glutamine, and may include other trace elements and buffers, etc., as cofactors.

The medium may also include an antibiotic. The medium may include various commercially available media known for animal cell culture, for example, Dulbecco's Modified Eagle's medium (DMEM), endothelial differentiation medium (EDM), minimal essential medium (MEM), basal medium eagle (BME), RPMI1640, F-10, F-12, α-minimal essential medium (α-MEM), Glasgow's minimal essential medium (G-MEM) and Iscove's modified Dulbecco's medium, etc., but is not limited thereto. In an embodiment, StemPro-34 SFM medium may be used.

In an embodiment, the concentration of CHIR99021 in the first culture medium composition may be 0.5 to 50 uM, 0.5 to 40 uM, 0.5 to 30 uM, 0.5 to 20 uM, 0.5 to 10 uM, 1 to 50 uM, 2 to 50 uM, 3 to 50 uM, 4 to 50 uM, 1 to 40 uM, 2 to 30 uM, 3 to 20 uM or 4 to 10 uM.

In an embodiment, the concentration of BMP4 in the first culture medium composition may be 5 to 500 ng/ml, 5 to 400 ng/ml, 5 to 300 ng/ml, 5 to 200 ng/ml, 5 to 100 ng/ml, 10 to 500 ng/ml, 20 to 500 ng/ml, 30 to 500 ng/ml, 40 to 500 ng/ml, 10 to 400 ng/ml, 20 to 300 ng/ml, 30 to 200 ng/ml, or 40 to 100 ng/ml.

In an embodiment, the concentration of VEGF in the first culture medium composition may be 5 to 500 ng/ml, 5 to 400 ng/ml, 5 to 300 ng/ml, 5 to 200 ng/ml, 5 to 100 ng/ml, 10 to 500 ng/ml, 20 to 500 ng/ml, 30 to 500 ng/ml, 40 to 500 ng/ml, 10 to 400 ng/ml, 20 to 300 ng/ml, 30 to 200 ng/ml, or 40 to 100 ng/ml.

In an embodiment, the concentration of bFGF in the first culture medium composition may be 1 to 1000 ng/ml, 1 to 900 ng/ml, 1 to 800 ng/ml, 1 to 700 ng/ml, 1 to 600 ng/ml, 1 to 500 ng/ml, 1 to 400 ng/ml, 1 to 300 ng/ml, 1 to 200 ng/ml, 10 to 1000 ng/ml, 20 to 1000 ng/ml, 30 to 1000 ng/ml, 40 to 1000 ng/ml, 10 to 900 ng/ml, 20 to 800 ng/ml, 30 to 700 ng/ml, 40 to 600 ng/ml, 40 to 500 ng/ml, 40 to 400 ng/ml, 40 to 300 ng/ml, 40 to 200 ng/ml, 10 to 200 ng/ml or 10 to 100 ng/ml.

In an embodiment, the concentration of a retinoic acid in the first culture medium composition may be 0.1 to 10 uM, 0.1 to 8 uM, 0.1 to 6 uM, 0.1 to 4 uM, 0.1 to 2 uM, 0.3 to 10 uM, 0.5 to 10 uM, 0.7 to 10 uM, 0.9 to 10 uM, 0.3 to 8 uM, 0.5 to 6 uM, 0.7 to 4 uM, or 0.9 to 2 uM.

In an embodiment, the concentration of SB-431542 in the first culture medium composition may be 1 to 100 uM, 1 to 80 uM, 1 to 60 uM, 1 to 40 uM, 1 to 20 uM, 3 to 100 uM, 5 to 100 uM, 7 to 100 uM, 9 to 100 uM, 3 to 80 uM, 5 to 60 uM, 7 to 40 uM, or 9 to 20 uM.

In some embodiments, the concentration of PVA in the first culture medium composition may be 0.01 to 1 %(w/v), 0.01 to 0.8 %(w/v), 0.01 to 0.6 %(w/v), 0.01 to 0.4 %(w/v), 0.01 to 0.2 %(w/v), 0.01 to 0.1 %(w/v), 0.03 to 1 %(w/v), 0.05 to 1 %(w/v), 0.07 to 1 %(w/v), 0.09 to 1 %(w/v), 0.03 to 0.8 %(w/v), 0.05 to 0.6 %(w/v), 0.07 to 0.4 %(w/v), or 0.09 to 0.2 %(w/v).

In an embodiment, the concentration of SCF in the first culture medium composition may be 5 to 500 ng/ml, 5 to 400 ng/ml, 5 to 300 ng/ml, 5 to 200 ng/ml, 5 to 100 ng/ml, 10 to 500 ng/ml, 20 to 500 ng/ml, 30 to 500 ng/ml, 40 to 500 ng/ml, 10 to 400 ng/ml, 20 to 300 ng/ml, 30 to 200 ng/ml, or 40 to 100 ng/ml.

In an embodiment, the concentration of SCF in the second culture medium composition may be 5 to 500 ng/ml, 5 to 400 ng/ml, 5 to 300 ng/ml, 5 to 200 ng/ml, 5 to 100 ng/ml, 10 to 500 ng/ml, 20 to 500 ng/ml, 30 to 500 ng/ml, 40 to 500 ng/ml, 10 to 400 ng/ml, 20 to 300 ng/ml, 30 to 200 ng/ml, or 40 to 100 ng/ml.

In an embodiment, the concentration of DKK1 in the second culture medium composition may be 3 to 300 ng/ml, 3 to 250 ng/ml, 3 to 200 ng/ml, 3 to 150 ng/ml, 3 to 100 ng/ml, 3 to 50 ng/ml, 5 to 300 ng/ml, 10 to 300 ng/ml, 15 to 300 ng/ml, 20 to 300 ng/ml, 25 to 300 ng/ml, 5 to 250 ng/ml, 10 to 200 ng/ml, 15 to 150 ng/ml, 20 to 100 ng/ml, or 25 to 50 ng/ml.

In an embodiment, the concentration of IL-3 in the second culture medium composition may be 2 to 200 ng/ml, 2 to 150 ng/ml, 2 to 100 ng/ml, 2 to 50 ng/ml, 5 to 200 ng/ml, 10 to 200 ng/ml, 15 to 200 ng/ml, 5 to 150 ng/ml, 10 to 100 ng/ml or 15 to 50 ng/ml.

In an embodiment, the concentration of fasudil in the second culture medium composition may be 1 to 100 uM, 1 to 80 uM, 1 to 60 uM, 1 to 40 uM, 1 to 20 uM, 3 to 100 uM, 5 to 100 uM, 7 to 100 uM, 9 to 100 uM, 3 to 80 uM, 5 to 60 uM, 7 to 40 uM, or 9 to 20 uM.

In an embodiment, the concentration of CSF-1 in the third culture medium composition may be 5 to 500 ng/ml, 5 to 400 ng/ml, 5 to 300 ng/ml, 5 to 200 ng/ml, 5 to 100 ng/ml, 10 to 500 ng/ml, 20 to 500 ng/ml, 30 to 500 ng/ml, 40 to 500 ng/ml, 10 to 400 ng/ml, 20 to 300 ng/ml, 30 to 200 ng/ml, or 40 to 100 ng/ml.

In an embodiment, the concentration of fasudil in the third culture medium composition may be 1 to 100 uM, 1 to 80 uM, 1 to 60 uM, 1 to 40 uM, 1 to 20 uM, 3 to 100 uM, 5 to 100 uM, 7 to 100 uM, 9 to 100 uM, 3 to 80 uM, 5 to 60 uM, 7 to 40 uM, or 9 to 20 uM.

The wording "positive or +" with respect to a cell label (marker) may refer to that the label is present in a greater amount or higher concentration compared to other cells that serve as a reference. A cell may be positive for a marker if the marker is present inside or on the surface of the cell such that the cell can be distinguished from one or more other cell types by using that marker. Also, it may indicate that a cell has the marker in an amount sufficient to produce a signal, for example, a signal from a cell measurement device, that is greater than a background value. For example, a cell can be detectably labeled with an antibody specific to CD56, and if the signal from this antibody is detectably greater than a control (for example, background value), the cell can be designated as "positive for CD56" or "CD56+". The term "negative or -" may indicate that the marker cannot be detected in comparison to the background value even when using an antibody specific to a particular cell surface marker. For example, if a cell cannot be detectably labeled with an antibody specific to CD3, the cell can be designated as "negative for CD3" or "CD3-".

In an embodiment, the macrophages differentiated by the medium composition for inducing differentiation from stem cells to macrophages of the present disclosure may have 50%, 60%, 70%, 80% or 90% or more of the cells in the total cell population being positive for CD86.

In an embodiment, the macrophages differentiated by the medium composition for inducing differentiation from stem cells to macrophages of the present disclosure may have 70%, 80% or 90% or more of the cells in the total cell population being positive for CD16.

In some embodiments, the stem cells may be pluripotent stem cells.

In an embodiment, the stem cells may be human embryonic stem cells (hESCs) or human induced pluripotent stem cells (hiPSCs).

The term "stem cell" as used herein refers to a cell that has the ability to self-renewal capability and the ability to differentiate into two or more cells, and can be classified as a totipotent stem cell, a pluripotent stem cell, and a multipotent stem cell.

The term "human pluripotent stem cells (hPSCs)" as used herein refer to stem cells that have self-renewal ability and are pluripotent or totipotent and can differentiate into cells of all tissues of a subject, and may include embryonic stem cells or induced pluripotent stem cells. "Embryonic stem cells" are cells that are cultured in vitro by extracting the inner cell mass from a blastocyst stage embryo just before the fertilized egg implants in the mother's uterus, and have self-reproduction capability with pluripotency or totipotency that can differentiate into cells of all tissues of an organism. In a broad sense, embryonic stem cells also includes embryoid bodies derived from embryonic stem cells. "Induced pluripotent stem cells" are cells that have been induced to have pluripotent differentiation ability through an artificial dedifferentiation process from differentiated cells, and are also called dedifferentiated stem cells. Artificial dedifferentiated processes may be performed by the use of viral-mediated or non-viral vectors using retroviruses and lentiviruses, by the introduction of non-viral-mediated dedifferentiating factors using proteins and cell extracts, etc., or by the dedifferentiating process using stem cell extracts, compounds, etc. Induced pluripotent stem cells have almost the same characteristics as embryonic stem cells, specifically showing similar cell morphology, similar gene and protein expression patterns, pluripotency in vitro and in vivo, forming teratomas, forming chimeric mice when inserted into mouse blastocysts, and enabling germline transmission of genes.

Another aspect provides a method of inducing differentiation from stem cells to macrophages, including: (i) inducing differentiation from stem cells to hematopoietic stem cells in the presence of a first culture medium composition including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof; (ii) inducing differentiation from hematopoietic stem cells to macrophages in the presence of a second culture medium composition including one or more selected from the group consisting of a stem cell factor (SCF), Dickkopf-related protein 1 (DKK1), interleukin 3 (IL-3), fasudil, and combinations thereof; and (iii) maturing macrophages in the presence of a third culture medium composition including one or more selected from the group consisting of a colony stimulating factor 1 (CSF-1), fasudil, and combinations thereof.

In an embodiment, during the induction of differentiation in process (i), the first culture medium composition may be replaced with a first culture medium composition having a different composition.

In an embodiment, process (i) may include culturing stem cells in a culture medium composition including BMP4, VEGF and bFGF for 5 to 20 days, 7 to 18 days, 9 to 16 days, 11 to 14 days, or 12 days.

In an embodiment, process (ii) may include culturing hematopoietic stem cells in a culture medium composition including bFGF for 3 to 10 days, 5 to 8 days, or 7 days.

In an embodiment, process (iii) may include culturing macrophages in a culture medium composition including fasudil for 3 to 10 days, 5 to 8 days, or 6 days.

In an embodiment, a pharmaceutical composition for preventing or treating cancer is provided, which includes, as an active ingredient, a macrophage or a cell population thereof differentiated by a method of inducing differentiation.

The "cancer" includes a tumor, a blood cancer, or a solid cancer. In an embodiment, the cancer may be at least one selected from the group consisting of glioma, gastrointestinal stromal tumor, leukemia, breast cancer, uterine cancer, cervical cancer, stomach cancer, colon cancer, prostate cancer, ovarian cancer, lung cancer, laryngeal cancer, rectal cancer, liver cancer, gallbladder cancer, pancreatic cancer, kidney cancer, skin cancer, bone cancer, muscle cancer, fatty cancer, fibroblast cancer, blood cancer, lymphoma, and multiple myeloma.

The term "prevention" as used herein refers to any act of inhibiting or delaying the occurrence of cancer by administering a composition according to the present disclosure.

The term "treatment" as used herein refers to or includes the alleviation, inhibition of progression, or prevention of a disease, disorder, or condition, or one or more symptoms thereof, and the term "active ingredient" or the term "pharmaceutically effective amount" may indicate any amount of the composition used in the course of practicing the disclosure provided herein sufficient to alleviate, inhibit progression, or prevent a disease, disorder, or condition, or one or more symptoms thereof.

The composition may additionally include other known immune antigen adjuvants, and the other immune antigen adjuvant may include one of monophosphoryl lipid A (MPL) and glucopyranosyl lipid adjuvant, formulated in a stable nano-emulsion of squalene oilin-water (GLA-SE).

The method of administration of the pharmaceutical composition is not particularly limited, but may be administered parenterally or orally, such as intravenously, subcutaneously, intraperitoneally, by inhalation, or topical application, depending on the intended method. The dosage varies depending on the patient's weight, age, gender, health status, diet, administration time, administration method, excretion rate, and disease severity. The daily dosage may be the amount of a therapeutic substance that is administered to a subject in need of treatment and is sufficient to treat the disease condition to be alleviated. The effective amount of a therapeutic agent varies depending on the specific compound, disease state and its severity, and the subject requiring treatment, which can be routinely determined by those skilled in the art. As a non-limiting example, the dosage for human use of the composition according to one aspect may vary depending on the patient's age, weight, gender, dosage form, health status, and severity of disease. For an adult patient weighing 70 kg, for example, about 1,000 to 10,000 cells/time, 1,000 to 100,000 cells/time, 1,000 to 1,000,000 cells/time, 1,000 to 10,000,000, 1,000 to 100,000,000 cells/time, 1,000 to 1,000,000,000 cells/time, 1,000 to 10,000,000,000 cells/time, may be administered in divided doses from once to several times per day at regular intervals, or may be administered multiple times at regular intervals.

The pharmaceutical composition may include pharmaceutically acceptable carriers and/or additives. For example, the pharmaceutical composition may include sterile water, saline solution, conventional buffers (such as a phosphoric acid, a citric acid, or other organic acids), stabilizers, salts, antioxidants (such as ascorbic acid), surfactants, suspending agents, isotonic agents, or preservatives. For local administration, the pharmaceutical composition may also include combinations with organic substances such as biopolymers, inorganic substances such as hydroxyapatite, specifically collagen matrices, polylactic acid polymers or copolymers, polyethylene glycol polymers or copolymers, and chemical derivatives thereof. When a pharmaceutical composition according to an embodiment is prepared in a dosage form suitable for injection, the immune cells or a substance that increases their activity may be dissolved in a pharmaceutically acceptable carrier or may be frozen in a dissolved solution state.

The pharmaceutical composition may appropriately include a suspending agent, a solubilizing agent, a stabilizer, an isotonic agent, a preservative, an anti-adsorption agent, a surfactant, a diluent, an excipient, a pH adjuster, a soothing agent, a buffer, a reducing agent, an antioxidant, etc., depending on the administration method or formulation. Pharmaceutically acceptable carriers and formulations suitable for the present disclosure, including those exemplified above, are described in detail in Remington's Pharmaceutical Sciences, 19th ed., 1995. The pharmaceutical composition may be prepared in unit dosage forms or placed in multi-dose containers by formulating with pharmaceutically acceptable carriers and/or excipients according to methods easily implemented by those of ordinary skill in the art to which the disclosure pertains. In this regard, the formulation may be in the form of a solution, suspension or emulsion in an oil or aqueous medium, or in the form of a powder, granules, tablets or capsules.

One aspect provides a kit for inducing differentiation from stem cells to macrophages, including: (i) a first culture medium composition for inducing differentiation from stem cells to hematopoietic stem cells, including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof; (ii) a second culture medium composition for inducing differentiation from hematopoietic stem cells to macrophages, including one or more selected from the group consisting of a stem cell factor (SCF), Dickkopf-related protein 1 (DKK1), interleukin 3 (IL-3), fasudil, and combinations thereof; and (iii) a third culture medium composition for macrophage maturation, including one or more selected from the group consisting of a colony stimulating factor 1 (CSF-1), fasudil, and combinations thereof.

### Effects of Invention

According to the culture medium composition for inducing differentiation from stem cells to macrophages according to an aspect, the differentiation efficiency to macrophages can be increased and the expression of macrophage-specific markers can be improved.

### Brief Description of Drawings

FIG. 1 shows a schematic diagram of the process of differentiating stem cells to macrophages.
FIG. 2 shows a result of observing changes in cell morphology during the differentiation process from stem cells to macrophages.
FIG. 3 shows the results of measuring the number of floating cells after inducing differentiation from stem cells to macrophages.
FIGS. 4 to 6 show the results of confirming markers specific to macrophages by using FACS.
FIG. 7 shows the results of measuring the phagocytosis of differentiated macrophages.

### Mode for Carrying out the Invention

Hereinafter, the present disclosure will be described in more detail through examples. However, these examples are intended to exemplify the present disclosure and the scope of the present disclosure is not limited to these examples.

Terms or words used in the specification and claims of the present disclosure are not to be construed as limited to their usual or dictionary meanings, and should be interpreted as meanings and concepts that conform to the technical idea of the present disclosure, based on the principle that the inventor can appropriately define the concept of the term in order to explain his or her own invention in the best manner.

Throughout the specification of the present disclosure, when a part is said to "include" a certain component, this does not exclude other components, but rather means that other components may be included, unless otherwise specifically stated.

Throughout the specification of the present disclosure, "A and/or B" refers to A or B, or A and B.

### Example 1: Human stem cell derived macrophage

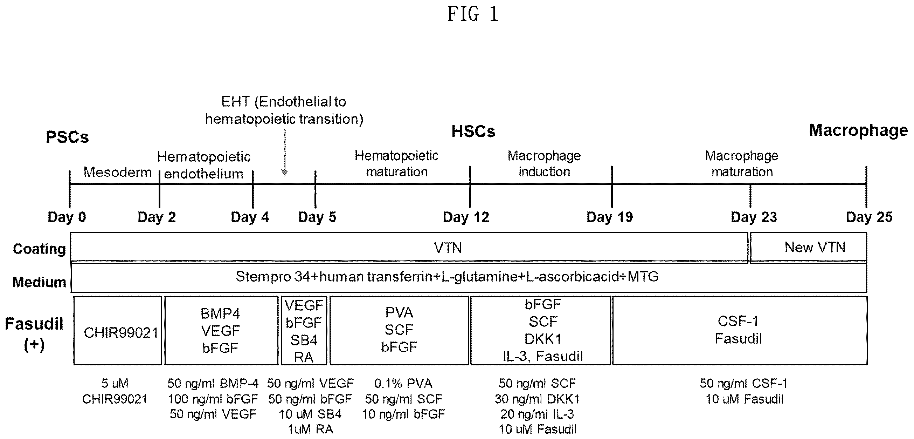

A schematic diagram of the process of differentiating stem cells to macrophages is shown in FIG. 1. Specifically, human pluripotent stem cells were cultured and differentiated into hematopoietic stem cells, and hematopoietic stem cells were cultured and differentiated to macrophages.

### 1.1.Differentiation into hematopoietic stem cells

Human pluripotent stem cells (hPSCs) were maintained in stemMACS-iPS brew media for 2 days. The differentiation basal culture medium included Stempro34-SFM (+stempro sup) + 200 ug/ml human Transferrin + 2 mM L-glutamine + 0.5 mM L-Ascobic acid + 0.45 mM MTG (1-thioglycerol) + 1% penicillin/streptomycin.

Cells were treated for 2 days with CHIR99021, known to induce mesenchyme, alone at a concentration of 5 uM. Additionally, cells were treated for 2 days with differentiation medium supplemented with 50 ng/ml of BMP4, 50 ng/ml of VEGF, and 100 ng/ml of bFGF. Subsequently, cells were cultured for the next day in medium supplemented with 50 ng/ml of VEGF, 50 ng/ml of bFGF, 10 uM of SB-431542, and 1 uM of retinoic acid. From the 5th to the 12th day after culture, cells were cultured in medium supplemented with 0.1% of PVA, 50 ng/ml of SCF, and 10 ng/ml of bFGF, with media replacement once a day.

### 1.2. Differentiation to macrophages

After differentiation into hematopoietic stem cells, cells were cultured for 7 days in medium supplemented with 10 ng/ml of bFGF, 50 ng/ml of SCF, 30 ng/ml of DKK1, 20 ng/ml of IL-3, and 10 uM of fasudil, with daily media replacement.

### 1.3.Maturation of macrophages

For an additional 4 days, cells were cultured in medium supplemented with 50 ng/ml of CSF-1 and 10 uM of fasudil. Subsequently, only floating cells in the same culture medium were isolated and cultured for 2 days in a culture dish coated with vitronectin (VTN).

### Comparative Example 1

### 1.1. Differentiation into hematopoietic stem cells

Human pluripotent stem cells (hPSCs) were maintained for 2 days in stemMACS-iPS brew media. The basic differentiation culture medium included Stempro34-SFM(+stempro sup) + 200 ug/ml human Transferrin + 2 mM L-glutamine + 0.5 mM L-Ascobic acid + 0.45 mM MTG(1-thioglycerol) + 1% penicillin/streptomycin.

Cells were treated with 5 uM of CHIR99021, 50 ng/ml of BMP4, and 50 ng/ml of VEGF for 2 days. Additionally, cells were treated for 2 days with differentiation medium supplemented with 50 ng/ml of BMP4, 50 ng/ml of VEGF, and 100 ng/ml of bFGF. Subsequently, cells were cultured for the next day in medium supplemented with 50 ng/ml of VEGF, 50 ng/ml of bFGF. From the 5th to the 12th day after culture, cells were cultured in medium supplemented with 50 ng/ml of VEGF, 50 ng/ml of SCF, and 10 ng/ml of bFGF, 30 ng/ml of DKK-1, 20 ng/ml of IL-3, 10 ng/ml of IL-6, with media replacement once a day.

### 1.2.Differentiation to macrophages

After differentiation into hematopoietic stem cells, cells were cultured for 7 days in medium supplemented with 10 ng/ml of bFGF, 50 ng/ml of SCF, 20 ng/ml of IL-3, and 10 ng/ml of IL-6, with daily media replacement.

### 1.3.Maturation of macrophages

For an additional 4 days, cells were cultured in medium supplemented with 50 ng/ml of CSF-1. Subsequently, only floating cells in the same culture medium were isolated and cultured for 2 days in a culture dish coated with vitronectin (VTN).

### Comparative Example 2

Macrophages were differentiated in the same manner as in Example 1, except that in section 1.2 of Example 1, culture medium supplemented with 10 ng/ml of IL-6 without addition of fasudil was used while keeping the addition concentrations of other components the same, and in section 1.3 of Example 1, culture medium without addition of fasudil was used while keeping the addition concentrations of other components the same.

### Example 1: Changes in cell morphology during differentiation to macrophages

Cell morphology was observed during the differentiation process of Example 1 and Comparative Example 1 to determine whether morphological changes occurred depending on the presence or absence of fasudil treatment.

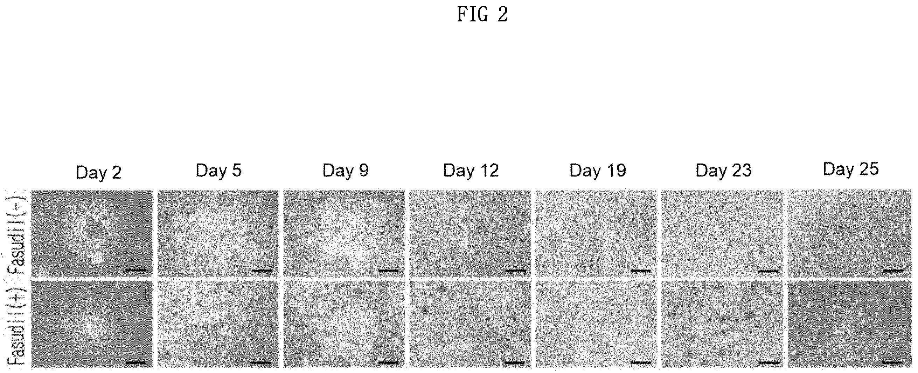

Specifically, cells were observed through a microscope on days 1, 5, 9, 12, 19, 23, and 25 after the start of differentiation. The results are shown in FIG. 2.

As shown in FIG. 2, in both Example 1 and Comparative Example 1, differentiation started from the center of the colony and expanded to the periphery, and the floating differentiated cells proliferated to form new colonies. Morphologically, it was found that there was no change depending on the presence or absence of fasudil treatment.

### Experimental Example 2: Confirmation of the number of floating cells

Since macrophages differentiated from human-derived hematopoietic stem cells are non-adherent floating cells, the number of differentiated cells increases as differentiation to macrophages progresses. Therefore, the number of floating cells was examined to determine the degree of differentiation to macrophages.

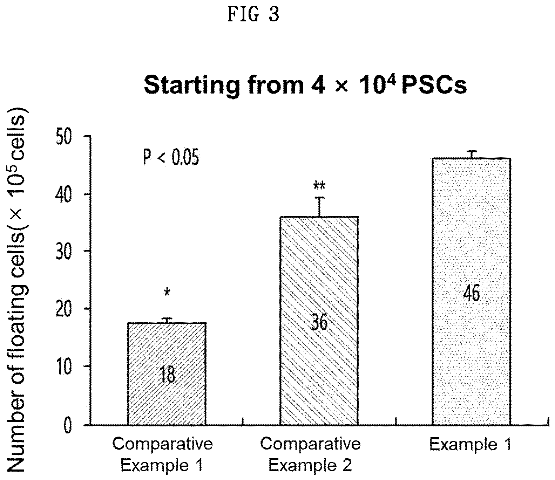

Specifically, after inducing differentiation using 4x10⁴ cells of hPSCs in Example 1, Comparative Example 1, and Comparative Example 2, the number of floating cells was measured on day 25 of differentiation. The results are shown in FIG. 3.

As shown in FIG. 3, the number of floating cells was 1.8x10⁶ cells in Comparative Example 2, 3.6x10⁶ cells in Comparative Example 1, and 4.6x10⁶ cells in Example 1, confirming that the number of floating cells was significantly increased in Example 1.

From the results, it was found that in Example 1, where differentiation occurred in medium containing fasudil, cells differentiated efficiently to macrophages.

### Experimental Example 3: Identification of macrophage-specific markers

The expression pattern of markers specific to macrophages in the cells obtained in Example 1, Comparative Example 1, and Comparative Example 2 was investigated. Specifically, confirmation was performed using fluorescence-activated cell sorting (FACS, BD FACSCalibur^{™}).

### 3.1. MHC 1 and MHC 2

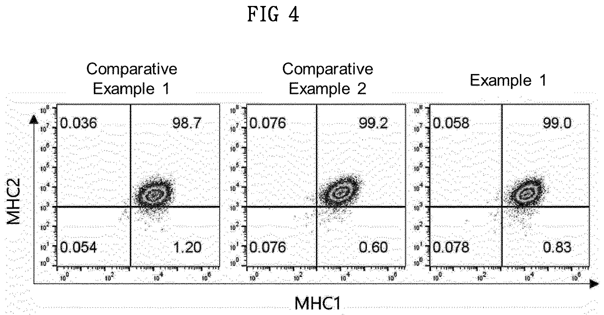

Results of cells expressing major histocompatibility complex (MHC) 1 and 2 confirmed through FACS are shown in FIG. 4.

MHC 2 is a protein expressed in antigen presenting cells (APC), and since MHC 2 is known to be present only in specific immune cells such as macrophages or B cells, it is indirect evidence of whether differentiation to macrophages has occurred.

As shown in FIG. 4, floating cells in all of Example 1, Comparative Example 1, and Comparative Example 2 were found to express both MHC 1 and 2.

From the results, it was found that differentiation to macrophages occurred in all of Example 1, Comparative Example 1, and Comparative Example 2.

### 3.2. CD11b and CD86

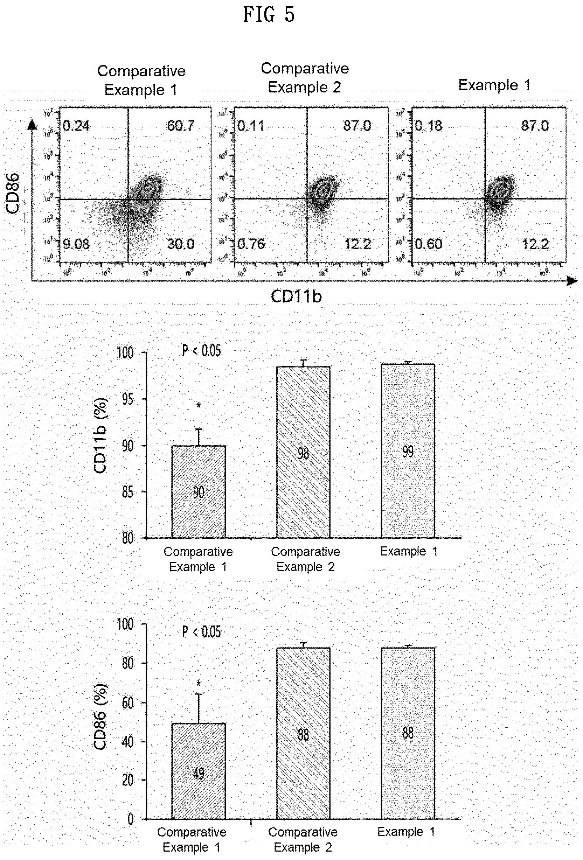

Results of cells expressing CD11b, a surface marker of macrophages, and CD86, which is expressed in antigen presenting cells, confirmed through FACS are shown in FIG. 5.

As shown in FIG. 5, the expression rate of CD11b was 90% in Comparative Example 2, 98% in Comparative Example 1, and 99% in Example 1. The expression rate of CD86 was 88%, 88%, and 49% in Example 1, Comparative Example 1, and Comparative Example 2, respectively.

From these results, it was found that when using medium supplemented with SB-431542, retinoic acid, and PVA during the differentiation process into hematopoietic stem cells, and using medium supplemented with DKK1 during the differentiation process to macrophages, the expression of macrophage-specific markers increased.

### 3.3. CX3CR1 and CD16

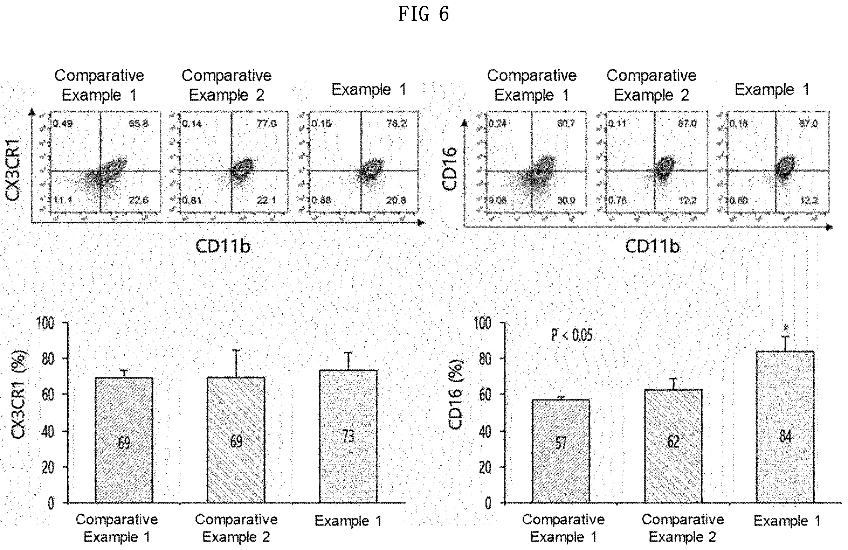

Results of cells expressing CX3CR1, which regulates macrophage function in inflammatory sites, and CD16, which is associated with antibody-dependent cell-mediated cytotoxicity (ADCC), confirmed through FACS are shown in FIG. 6.

As shown in FIG. 6, the expression rate of CX3CR1 was 73%, 69%, and 69% in Example 1, Comparative Example 1, and Comparative Example 2, respectively, with no statistically significant difference. The expression rate of CD16 was 84%, 62%, and 57% in Example 1, Comparative Example 1, and Comparative Example 2, respectively, confirming that it was significantly higher in Example 1 where fasudil was treated.

From these results, it was found that when using medium supplemented with fasudil during the differentiation process to macrophages, the expression of macrophage-specific markers increased.

### Experimental Example 4: Measurement of phagocytosis

Phagocytosis of macrophages obtained from Example 1 and Comparative Example 1 was confirmed.

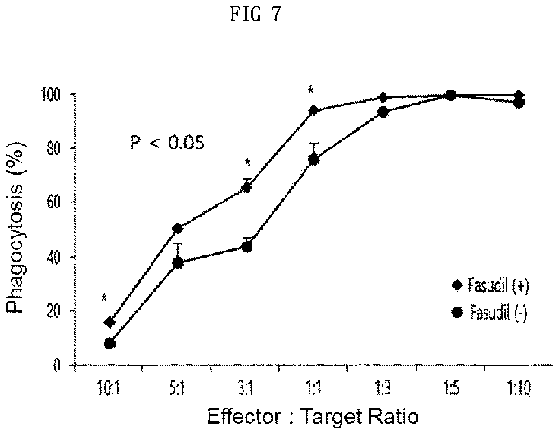

Specifically, macrophages (effectors) obtained from Example 1 and Comparative Example 1 were co-cultured with mouse thymus cells (targets, fluorescently stained) at various E:T (effector:target) ratios. After co-culture, cells were collected and phagocytosis of macrophages was measured by measuring cells that simultaneously expressed macrophage-specific markers (CD11b) and fluorescence that had been stained on target cells. The results are shown in FIG. 7.

As shown in FIG. 7, higher phagocytosis function was confirmed in Example 1 treated with fasudil compared to Comparative Example 1 where fasudil was not treated.

From these results, it was found that when using medium supplemented with fasudil during the differentiation process to macrophages, phagocytosis of macrophages increased.

## Claims

1. A culture medium composition for inducing differentiation from stem cells into macrophages, the culture medium composition comprising:
(i) a first culture medium composition for inducing differentiation from stem cells to hematopoietic stem cells, including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof;
(ii) a second culture medium composition for inducing differentiation from hematopoietic stem cells to macrophages, including one or more selected from the group consisting of a stem cell factor (SCF), Dickkopf-related protein 1 (DKK1), interleukin 3 (IL-3), fasudil, and combinations thereof; and
(iii) a third culture medium composition for macrophage maturation, including one or more selected from the group consisting of a colony stimulating factor 1 (CSF-1), fasudil, and combinations thereof.

2. The culture medium composition of claim 1, wherein, in the first culture medium composition, the concentration of CHIR99021 is 0.5 to 50 uM, the concentration of BMP4 is 5 to 500 ng/ml, the concentration of VEGF is 5 to 500 ng/ml, the concentration of bFGF is 1 to 1000 ng/ml, the concentration of retinoic acid is 0.1 to 10 uM, the concentration of SB-431542 is 1 to 100 uM, the concentration of PVA is 0.01 to 1 %(w/v), and the concentration of SCF is 5 to 500 ng/ml.

3. The culture medium composition of claim 1, wherein, in the second culture
medium composition, the concentration of SCF is 5 to 500 ng/ml, the concentration of DKK1 is 3 to 300 ng/ml, the concentration of IL-3 is 2 to 200 ng/ml, and the concentration of fasudil is 1 to 100 uM.

4. The culture medium composition of claim 1, wherein, in the third culture
medium composition, the concentration of CSF-1 is 5 to 500 ng/ml, and the concentration of fasudil is 1 to 100 uM.

5. The culture medium composition of claim 1, wherein
50% or more of cells in the total cell population of the differentiated macrophages are positive for CD86.

6. The culture medium composition of claim 1, wherein
70% or more of cells in the total cell population of the differentiated macrophages are positive for CD16.

7. The culture medium composition of claim 1, wherein
the stem cells are human embryonic stem cells (hESCs) or human induced pluripotent stem cells (hiPSCs).

8. A method of inducing differentiation from stem cells to macrophages, the method comprising:
(i) inducing differentiation from stem cells into hematopoietic stem cells in the presence of a first culture medium composition including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof;
(ii) inducing differentiation of hematopoietic stem cells to macrophages in the presence of a second culture medium composition including one or more selected from the group consisting of a stem cell factor (SCF), Dickkopf-related protein 1 (DKK1), interleukin 3 (IL-3), fasudil, and combinations thereof; and
(iii) maturing macrophages in the presence of a third culture medium composition including one or more selected from the group consisting of a colony stimulating factor 1 (CSF-1), fasudil, and combinations thereof.

9. The method of claim 8, wherein
process (i) comprises culturing stem cells for 5 to 20 days in a culture medium composition including BMP4, VEGF and bFGF.

10. The method of claim 8, wherein
process (ii) comprises culturing hematopoietic stem cells in a culture medium composition including bFGF for 3 to 10 days.

11. The method of claim 8, wherein
process (iii) comprises culturing macrophages in a culture medium composition including fasudil for 3 to 10 days.

12. A pharmaceutical composition for preventing or treating cancer, comprising
a macrophage or a cell population thereof differentiated by the method according to claim 8 as an active ingredient.

13. The pharmaceutical composition of claim 12, wherein
the cancer comprises at least one selected from the group consisting of glioma, gastrointestinal stromal tumor, leukemia, breast cancer, uterine cancer, cervical cancer, stomach cancer, colon cancer, prostate cancer, ovarian cancer, lung cancer, laryngeal cancer, rectal cancer, liver cancer, gallbladder cancer, pancreatic cancer, kidney cancer, skin cancer, bone cancer, muscle cancer, fatty cancer, fibroblast cancer, blood cancer, lymphoma, and multiple myeloma.

14. A kit for inducing differentiation from stem cells to macrophages, the kit
comprising:
(i) a first culture medium composition for inducing differentiation from stem cells to hematopoietic stem cells, including one or more selected from the group consisting of CHIR99021, bone morphogenetic protein 4 (BMP4), a vascular endothelial growth factor (VEGF), a basic fibroblast growth factor (bFGF), a retinoic acid, SB-431542, poly vinyl alcohol (PVA), a stem cell factor (SCF), and combinations thereof;
(ii) a second culture medium composition for inducing differentiation from hematopoietic stem cells to macrophages, including one or more selected from the group consisting of a stem cell factor (SCF), Dickkopf-related protein 1 (DKK1), interleukin 3 (IL-3), fasudil, and combinations thereof; and
(iii) a third culture medium composition for macrophage maturation, including one or more selected from the group consisting of a colony stimulating factor 1 (CSF-1), fasudil, and combinations thereof.

15. A method of preventing or treating cancer, comprising administering, to a
subject in need thereof, an effective amount of a macrophage or a cell population thereof differentiated by the method according to claim 8.

16. Use of a macrophage or a cell population thereof differentiated by the
method according to claim 8 for use in the preparation of a pharmaceutical formulation for the prevention or treatment of cancer.
